# EUROPEAN PATENT APPLICATION

(11) **EP 2 000 141 A1**
(43) Date of publication of application: **10.12.2008**
(21) Application number: 08009204.2
(22) Date of filing: 20.03.2003
(51) Int. Cl.: A61K 31/55, A61P 25/18, C07D 281/16

(54) **Methods for preparing crystalline quetiapine hemifumarate**

(30) Priority: 20.03.2002 US 365913 P; 29.01.2003 US 443585 P
(62) Divisional of application: 03721434.3
(71) Applicant: Teva Pharmaceutical Industries Ltd., 49131 Petah Tiqva (IL)
(72) Inventor: Diller, Dov, 96464 Jerusalem (IL); Lifshitz-Liron, Revital, 46322 Herzlia (IL); Kovalevski-Ishai, Eti, Netanya 42255 (IL); Lidor-Hadas, Rami, 44242 Kafar-Saba (IL); Wizel, Shlomit, 49742 Petah Tiqva (IL)
(74) Representative: Wong, Kit Yee

(57) **Abstract**

The present invention relates to methods for the preparation of crystalline quetiapine hemifumarate.

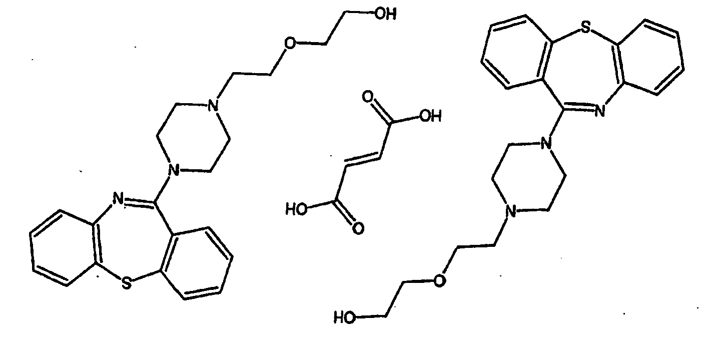

## Description

### FIELD OF THE INVENTION

The present invention relates to novel crystalline forms of quetiapine hemifumarate and methods of making them.

### BACKGROUND OF THE INVENTION

Many pharmaceutically active organic compounds can crystallize with more than one type of molecular packing with more than one type of internal crystal lattice. The respective resulting crystal structures can have, for example, different unit cells. This phenomenon - identical chemical structure but different internal structure - is referred to as polymorphisim and the species having different molecular structures are referred to as polymorphs.

Many pharmacologically active organic compounds can also crystallize such that a second, foreign molecules, especially solvent molecules, are regularly incorporated into the crystal structure of the principal pharmacologically active compound. This phenomenon is referred to as pseudopolymorphism and the resulting structures as pseudopolymorphs. When the second molecule is a solvent molecule, the pseudoploymorphs can be referred to as solvates.

The discovery of a new polymorph or pseudopolymorph of a pharmaceutically useful compound provides an opportunity to improve the performance characteristics of a pharmaceutical product. It enlarges the repertoire of materials that a formulation scientist has available for designing, for example, a pharmaceutical dosage form of a drug with a targeted release profile or other desired characteristic. It is clearly advantageous when this repertoire is enlarged by the discovery of new polymorphs or pseudopolymorphs of a useful compound. For a general review of polymorphs and the pharmaceutical applications of polymorphs *see* G.M. Wall, Pharm Manuf. 3, 33 (1986); J.K. Haleblian and W. McCrone, J. Pharm. Sci., 58, 911 (1969); and J.K. Haleblian, J. Pharm. Sci., 64, 1269 (1975), all of which are incorporated herein by reference.

Polymorphs and pseudopolymorphs can be influenced by controlling the conditions under which the compound is obtained in solid form. Solid state physical properties that can differ from one polymorph to the next include, for example, the flowability of the milled solid. Flowability affects the ease with which the material is handled during processing into a pharmaceutical product. When particles of the powdered compound do not flow past each other easily, a formulation specialist must take that fact into account in developing a tablet or capsule formulation, which may necessitate the use of glidants such as colloidal silicon dioxide, talc, starch or tribasic calcium phosphate.

Another important solid state property of a pharmaceutical compound that can vary from one polymorph or pseudopolymorph to the next is its rate of dissolution in aqueous media, e.g., gastric fluid. The rate of dissolution of an active ingredient in a patient's stomach fluid can have therapeutic consequences since it imposes an upper limit on the rate at which an orally-administered active ingredient can reach the patient's bloodstream. The rate of dissolution is also a consideration in formulating syrups, elixirs and other liquid medicaments. The solid state form of a compound may also affect its behavior on compaction and its storage stability.

These practical physical characteristics are influenced by the conformation and orientation of molecules in the unit cell, which characterize a particular polymorphic or pseudopolymorphic form of a substance. The polymorphic form may give rise to thermodynamic properties different from those of the amorphous material or another polymorphic form. Thermodynamic properties can be used to distinguish between polymorphs and pseudopolymorphs. Thermodynamic properties that can be used to distinguish between polymorphs and pseudopolymorphs can be measured in the laboratory by such techniques as capillary melting point, thermogravimetric analysis (TGA), differential scanning calorimetry (DSC), and differential thermal analysis (DTA).

A particular polymorph or pseudopolymorph can also possess distinct spectroscopic properties that may be detectable by, for example, solid state ¹³C NMR spectroscopy and infrared (IR) spectroscopy. This is particularly so in the case of pseudopolymorphs that are solvates because of the presence of absorptions or resonanaces due to the second, foreign molecule.

X-ray crystallography on powders (powder diffractometry) can be used to obtain x-ray diffraction diagrams that reveal information on the crystal structure of different polymorphs and pseudopolymorphs.

Quetiapine hemifumarate is a psychoactive organic compound that is an antagonist for multiple neurotransmitter receptors in the brain. Quetiapine hemifumarate is useful for treating, among other things, schizophrenia. Quetiapine hemifumarate can be made, for example, as taught in United States Patent 4,879,288, incorporated in its entirety herein by reference. X-ray diffraction data and Fourier transform IR data for quetiapine hemifumarate obtained by the procedure therein taught are presented below.

The structure of quetiapine, 2-[2-(4-dibenzo*[b,f]*][1,4]thiazepin-11-yl-1-piperazinyl)ethoxy]-ethanol fumarate (2:1), is shown below (I).

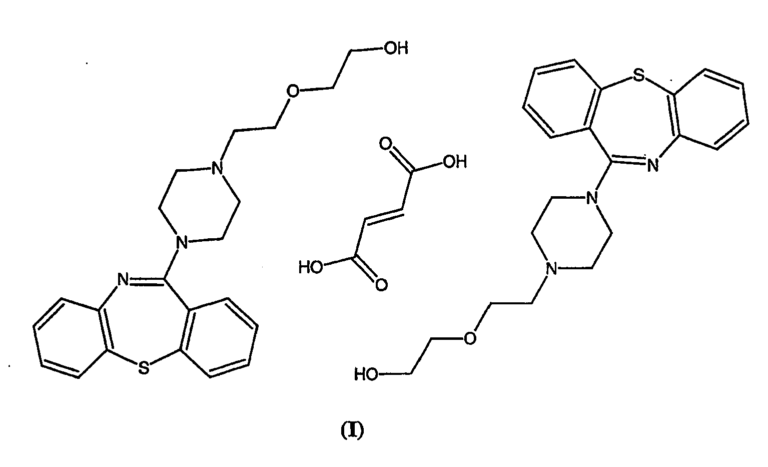

Applicants have discovered that quetiapine hemifumarate is an example of an organic compound that can exist in different crystal forms, different from the material obtained according to the teachings of the '288 patent and having useful properties. In particular, Applicants have discovered that treatment of quetiapine hemifumarate with a treating solvent can produce novel pseudopolymorphic forms of quetiapine hemifumarate.

In Applicants' hands, the methods of the '288 patent yield a crystalline form, which Applicants denote as form I, different from the crystal forms of the present invention.

### SUMMARY OF THE INVENTION

In one aspect, the present invention relates to a novel crystalline form of quetiapine hemifumarate that can be characterized by any one of: x-ray reflections at 7.8°, 11.9°, 12.5°, 15.7°, 23.0°, and 23.4°, ± 0.2° 2θ; absorption bands in FTIR spectroscopy at 639, 1112, 1395, 1616, 1711, and 3423 cm⁻¹; or a differential scanning calorimetric thermogram with endothermic peaks at about 130°C and at about 166°C. This crystalline form is denominated II.

This crystal form can exist as a solvate, especially a chloroform or methylene chloride (dichloromethane) solvate. Thus, in another aspect, the present invention relates to a crystalline dichloromethane solvate. characterized by x-ray reflections at 7.8°, 11.9°, 12.5°, 15.7°, 23.0°, and 23.4°, ± 0.2° 2θ, absorption bands in FTIR at 639, 1112, 1395, 1616, 1711, and 3423 cm⁻¹, and a thermogram in differential scanning calorimetry having endothermic peaks at about 130°C and about 166°C.

In another aspect, the present invention relates a solvate with chloroform characterized by x-ray reflections at 7.8°, 11.9°, 12.5°, 15.7°, 23.0°, and 23.4°, ± 0.2° 2θ, absorption bands in FTIR at 639, 1112, 1395, 1616, 1711, and 3423 cm⁻¹, and a thermogram in differential scanning calorimetry having endothermic peaks at about 130°C and about 166°C.

In another embodiment, the present invention relates to a method of making crystalline quetiapine hemifumarate having at least one characteristic of form II including the steps of: combining quetiapine hemifumarate and a treating solvent selected from chloroform and methylene chloride; refluxing the combination; cooling the combination after reflux, especially to a temperature of about room temperature; and isolating the crystalline form of quetiapine hemifumarate.

In a further aspect, the present invention relates to a method of making crystalline quetiapine hemifumarate having at least one characteristic of form II including the steps of: treating quetiapine hemifumarate with a treating solvent selected from chloroform and methylene chloride, and isolating the crystalline quetiapine hemifumarate having at least one characteristic of form II. The treating can be by a reflux method that includes the steps of: combining quetiapine hemifumarate and treating solvent; refluxing the combination; cooling the combination after reflux; and isolating the crystalline quetiapine hemifumarate having at least one characteristic of form II. The treating can also be by a solution method that includes the steps of: providing a solution of quetiapine hemifumarate in a dipolar aprotic solvent at a dissolution temperature, especially about 80°C; combining the solution with a treating solvent selected from chloroform and methylene chloride; cooling the combination to a temperature of about 20° C or less.

In yet another embodiment, the present invention relates to a novel crystalline form of quetiapine hemifumarate, which we denominate form III, that can be characterized by any one of: x-ray reflections at about 8.9°, 11.8°, 15.3°, 19.4°, 23.0°, and 23.4°, ± 0.2° 2θ, absorption bands in FTIR spectroscopy at 748, 758, 1402, 1607, 1715, and 2883 cm⁻¹, or a DSC thermogram with endothermic peaks at about 111°C, about 142°C, and about 167°C.

This crystal form can also exist as a solvate, especially a chloroform solvate. Thus, in another aspect, the present invention relates to quetiapine hemifumarate as a chloroform solvate characterized by x-ray reflections at about 8.9°, 11.8°, 15.3°, 19.4°, 23.0°, and 23.4°, ± 0.2° 2θ, and absorption bands in FTIR at 748, 758, 1402, 1607, 1715, and 2883 cm⁻¹.

In another aspect, the present invention relates to a method of making a crystalline form of quetiapine hemifumarate having one characteristic of form III, especially as its chlorofom solvate which method includes the steps of: providing a combination of quetiapine hemifumarate and a dipolar aprotic solvent at a temperature of about 80° C; mixing the combination with chloroform; optionally holding the mixture for a holding time, especially a holding time of about 14 hours; cooling the resulting mixture; and isolating the quetiapine hemifumarate form III chloroform solvate from the mixture. In still a further aspect, the present invention relates to a method of making prior art crystalline form I of quetiapine hemifumarate, which method includes the steps of: providing a solution at about 80° C of quetiapine hemifumarate in a solvent selected from the group consisting of water, alkanol, especially isopropyl alcohol or methanol, and dipolar aprotic solvents, especially dimethylsulfoxide, dimethylformamide, dimethylacetamide and 1-methyl-2-pyrrolidone and the anti-solvent is selected from the group consisting of water, ethylacetate, dichloromethane, toluene, acetone, acetonitrile, isobutanol, ethylacetate, isopropylacetate or methyl *tert*-butyl ether; combining the solution with an anti-solvent whereby a suspension is obtained; and isolating quetiapine hemifumarate form I from the suspension.

In still a further aspect, the present invention relates to a method of making quetiapine hemifumarate form I including the steps of: providing a solution at about 80° C of quetiapine hemifumarate in a solvent selected from the group consisting of alkanols, and a combination of a dipolar aprotic solvent and water; cooling the solution to a temperature of about 20° C or less; and isolating the quetiapine hemifumarate form I from the mixture.

In another aspect, the present invention relates to micronized quetiapine hemifumarate in form II, form III, or any solvate, especially a methylene chloride or chloroform solvate, of either of them.

In yet a further aspect, the present invention relates to a pharmaceutical composition that includes quetiapine hemifumarate having at least one characteristic of form II, form III, or a methylene chloride or chloroform solvate thereof, and at least one pharmaceutically acceptable excipient.

In yet still a further aspect, the present invention relates to a method of treating a mammal in need of treatment with quetiapine hemifumarate including the step of administering to such mammal a therapeutically effective amount of a pharmaceutical composition including quetiapine hemifumarate having at least one characteristic of form II, form III, or a methylene chloride or chloroform solvate thereof, and at least one pharmaceutically acceptable excipient.

In yet a further aspect, the present invention relates to a method of post-treating a crystalline form of quetiapine hemifumarate, especially form I, selected from a post-suspension method and a post-crystallization method.

The post-suspension method includes the steps of combining the isolated quetiapine hemifumarate form I with a post-suspending solvent selected from dialkyl ketones, aromatic hydrocarbons, cyanoalkanes, dialkyl ethers, and methylene chloride; refluxing the combination for a reflux time; cooling the combination to ambient temperature; optionally agitating the suspension for an agitating time; and isolating quetiapine hemifumarate form I. Examples of post-suspension solvents include acetone, toluene, acetonitrile, dichloromethane, and methyl *t*-butyl ether.

The post-crystallization method includes the steps of: a) refluxing a solution of the isolated quetiapine hemifumarate form I in a post-crystallization solvent selected from lower alkanols, cyclic ethers, ethyl acetate, and water for a reflux time; cooling the solution to ambient temperature whereby a suspension is formed, optionally agitating the suspension for an agitation time; and isolating the quetiapine hemifumarate form I. Examples of post-crystallization solvents include water, ethanol, isopropanol, 1-propanol, 1-butanol, 2-butanol, ethyl acetate, tetrahydrofuran, and 1,4-dioxane.

### BRIEF DESCRIPTION OF THE DRAWINGS

Figure 1 shows the x-ray diffraction diagram of quetiapine hemifumarate form II as its chloroform solvate.
Figure, 2 shows the FTIR spectrum of quetiapine hemifumarate form II as its chloroform solvate.
Figure 3 shows the DSC thermogram of quetiapine hemifumarate as its form II chloroform solvate.
Figure 4 shows the TGA trace of quetiapine hemifumarate as its form II chloroform solvate.
Figure 5 shows the x-ray diffraction diagram of quetiapine hemifumarate as its form II dichloromethane solvate.
Figure 6 shows the x-ray diffraction diagram of quetiapine hemifumarate form III as its chloroform solvate.
Figure 7 shows the FTIR spectrum of quetiapine hemifumarate form III as its chloroform solvate.
Figure 8 shows the DSC thermogram of form III.

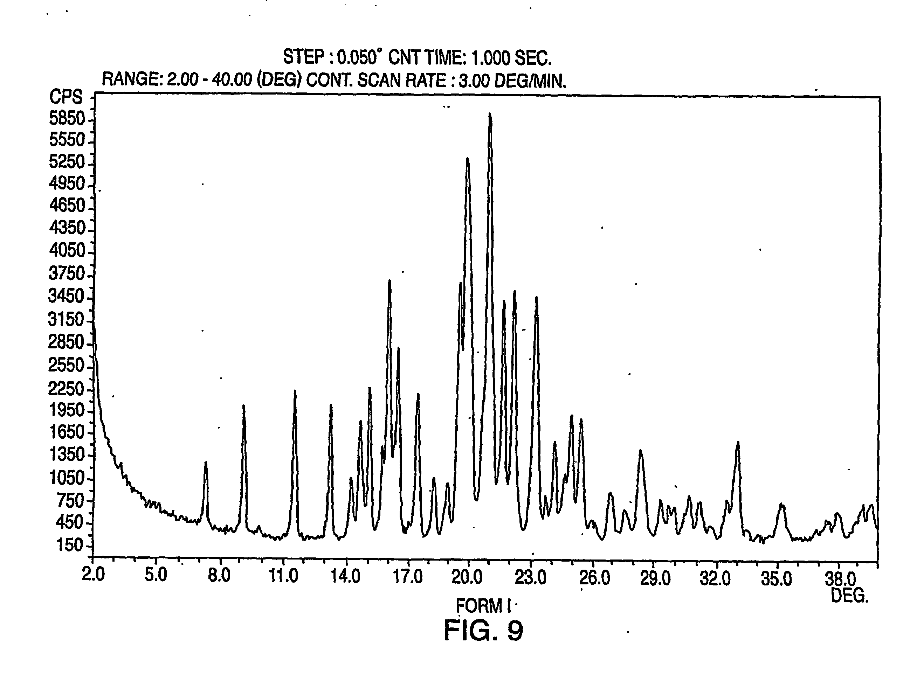

Figure 9 shows the x-ray diffraction diagram of quetiapine hemifumarate form I as taught by the '288 patent.

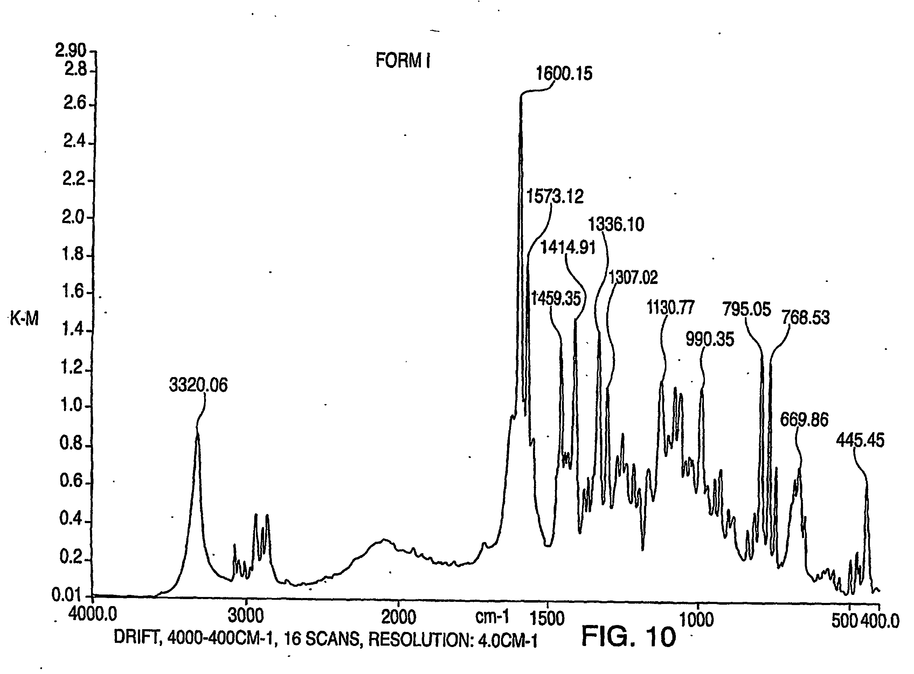

Figure 10 shows the FTIR spectrum of quetiapine hemifumarate form I as taught by the '288 patent.

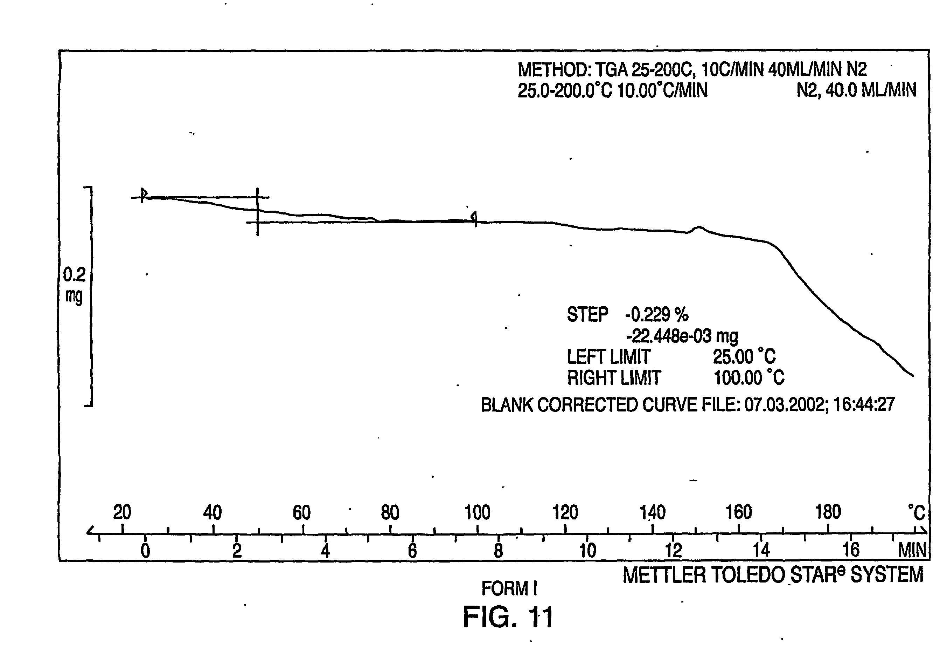

Figure 11 shows the TGA trace of quetiapine hemifumarate form I as taught by the '288 patent.

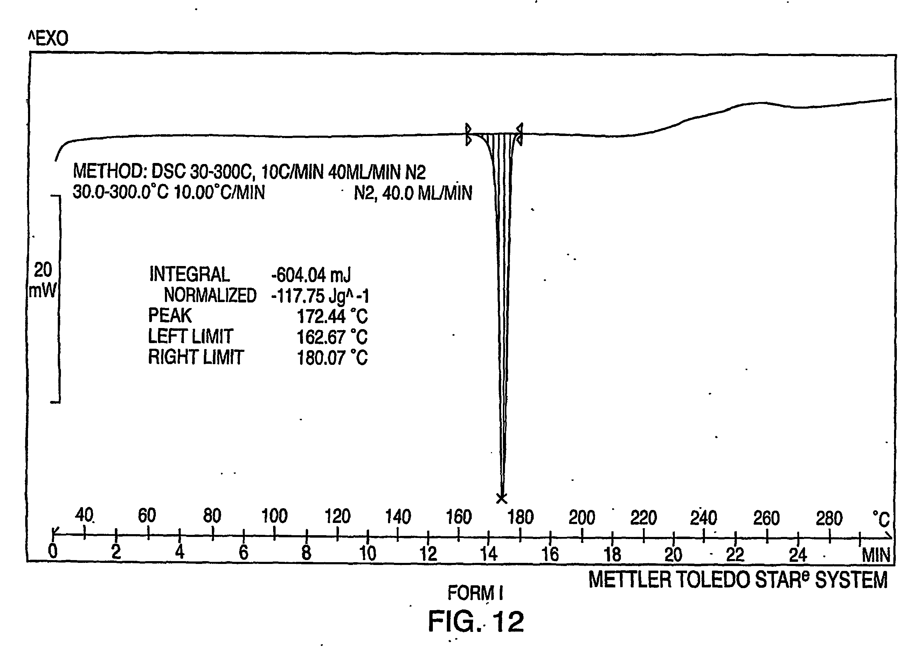

Figure 12 shows the DSC thermogram of quetiapine hemifumarate form I as taught by the '288 patent.

### DETAILED DESCRIPTION OF THE INVENTION

The present invention provides novel crystalline forms of quetiapine hemifumarate ("QTP") and methods for making them. As used herein and unless otherwise indicated, quetiapine hemifumarate and QTP refer to 2-[2-(4-dibenzo[*b*,*f*][1,4]thiazepin-11-yl-1-piperazinyl)ethoxy]-ethanol fumarate (2:1) salt.

The novel crystalline forms of quetiapine hemifumarate of the present invention can be characterized by any one of x-ray diffraction (XRD) or FTIR spectroscopy or differential scanning calorimetry (DSC). The novel crystalline forms of the present invention can exist as solvates, especially solvates with chlorinated hydrocarbons. Upon heating, the solvates lose solvating solvent. Release (loss) of the solvating solvent can be detected by thermogravimetric analysis (TGA).

As used herein, quetiapine hemifumarate refers to quetiapine hemifumarate in any crystalline form (polymorph or pseudopolymorph), or in an amorphous form, or any combination of these. One of skill in the art would appreciate that the polymorphs and pseudopolymorphs of the present invention can be selectively obtained generally through crystallization with different recrystallization solvent systems. The starting material can be quetiapine, quetiapine hemifumarate or any quetiapine hemifumarate hydrate or lower alcohol solvate. The starting quetiapine hemifumarate can also be in an amorphous or any crystalline crystal form.

A method for the synthesis of quetiapine, 11-piperazinyl dibenzo[*b,f*][1,4] thiazepinehydrochloride, is discussed, *inter alia*, in United States Patent No. 4,879,288, (the '288 patent) which is incorporated herein in its entirety by reference. In the preparation of quetiapine as described, 2-(2-chloroethoxy)ethanol is reacted with 11-piperazinyl dibenzo[b,f][1,4] thiazepinehydrochloride to form 2-(2-(4-dibenzo[*b,f*][1,4]thiazepin-11-yl-1-piperazinyl)ethoxy)ethanol. Reaction time as long as 50 hours can be required. *(See, e.g.,* '288 patent.) In Applicants' hands, the methods of the '288 patent yield a crystalline form, which Applicants denote as form I, different from the crystal forms of the present invention.

As used in connection with the present invention, x-ray diffraction (XRD) refers to x-ray diffraction by the powder diffraction technique. X-ray powder diffraction analysis was performed using a Scintag powder diffractometer with variable goniometer, a Cu source, and a solid state detector. A standard round aluminum sample holder with zero background quartz plate was used. All powder X-ray diffraction patterns were obtained by methods known in the art using 0.05 degree step size over the scanning range from 4° to 30°, or from 2° to 40° 2θ at 3° per minute. Copper radiation of λ = 1.5418 Å was used. Reflections are reported as peak maxima in the intensity vs. 2θ plots, and are subject to the normal experimental error (uncertainty). Wet samples were promptly analyzed "as is," i.e., without drying or grinding prior to the analysis.

In the present invention, infrared (IR) spectra were obtained by the diffuse reflectance technique of Fourier transform IR spectroscopy (FTIR) using a Perkin-Elmer One FTIR Spectrometer.

Differential scanning calorimetry (DSC) and thermogravimetric analysis (TGA) thermograms presented herein were obtained by methods known in the art. Differential scanning calorimetric (DSC) analysis was performed with a Mettler Toledo DSC 821^{e} calorimeter. Samples of about 3 to about 5 milligrams, held in a vented (3-hole) crucible, were analyzed at a heating rate of 10° per minute.

Thermogravimetric analysis (TGA) was performed using a Mettler TG50 thermobalance. TGA traces reflect transitions that involve either a loss or gain of mass. Samples of 7 to 15 milligrams were analyzed at a heating rate of 10°C per minute in nitrogen atmosphere.

As used herein, LOD refers to loss on drying as determined by TGA.

As used herein, ambient temperature means a temperature from about 20°C to about 25°C.

As used herein, alkanol refers to compounds of the general formula ROH, where R is a linear or branched alkyl group having up to 6 carbon atoms.

As used herein in connection with a measured quantity, the term, "about," refers to the normal variation in that quantity as expected by the skilled artisan making the measurement and exercising a level of care commensurate with the objective of the measurement and the precision of the measuring equipment.

As used herein, the phrase, "having at least one characteristic of quetiapine hemifumarate form '#,'" refers to a crystalline form of quetiapine hemifumarate that exhibits at least the characteristic powder x-ray diffraction (XRD) reflections (peaks) or the characteristic absorption bands in FTIR spectroscopy or the DSC thermograms of form "#."

Some processes of the present invention involve crystallization out of a particular solvent. One skilled in the art knows that some of the conditions concerning crystallization can be modified without affecting the form of the polymorph obtained. For example, when mixing quetiapine hemifumarate in a solvent to form a solution, warming of the mixture can be necessary to completely dissolve the starting material. If warming does not clarify the mixture, the mixture can be diluted or filtered.

The conditions can also be changed to induce precipitation. A preferred way of inducing precipitation from solution is to reduce the solubility of the solute in the solvent by, for example, cooling the solution.

Alternatively, an anti-solvent can be added to a solution to decrease solubility for a particular compound, thus resulting in precipitation.

In one embodiment, the present invention provides novel crystalline forms of quetiapine hemifumarate, in particular crystalline forms that are solvates in which the molecules of solvent, derived from a treating solvent and referred to as solvating solvent, are incorporated into the crystal structure. Solvating solvent can be removed by, for example, heating at atmospheric or reduced pressure.

According to the present invention, solvates (pseudopolymorphs) are prepared by treating quetiapine hemifumarate with a treating solvent as described below. Preferred treating solvents are linear or branched chlorinated hydrocarbons having the general formula CₙH(₂ₙ₋ₘ₊₂)Clₘ, where n is 1 to 4 and m is from 1 up to 2n+2. Dichloromethane and chloroform are particularly preferred treating solvents.

In accordance with the present invention, quetiapine hemifumarate pseudopolymorphs are made by treating quetiapine hemifumarate with a treating solvent. Treating can be in solution in a dipolar aprotic solvent. The treating can also be by a reflux method in which quetiapine hemifumarate is suspended in treating solvent at reflux. Refluxing and suspension can be carried out in a variety of apparatus or equipment that will be apparent to skilled artisan and routiner alike, including beakers, flasks, and tank reactors. Required agitation can be provided by mechanical or magnetic stirrers and agitators.

Quetiapine hemifumarate form II as its chlorinated hydrocarbon solvates can be made by treating quetiapine hemifumarate with a treating solvent that is a chlorinated hydrocarbon. The relative amount of treating solvent is not critical. Generally, between about 20 mL and about 60 mL of treating solvent are used for each gram of quetiapine hemifumarate to be treated. However, the routiner will know to adjust the proportions depending on, for example, the equipment to be used for treating.

Similarly, the time of treatment is not critical but can vary from about 1 to about 48 hours, with 2 to 24 hours being typical.

The treatment can be by a reflux method or by a solution method. In the reflux method, quetiapine hemifumarate is refluxed with a chlorinated hydrocarbon treating solvent for a reflux time. The skilled artisan will know to adjust the reflux time according to the relative amounts of quetiapine hemifumarate, treating solvent and the equipment used. The reflux time can be 6 hours or more.

In other embodiments, quetiapine hemifumarate form II solvates can be made by the solution method. In the solution method, quetiapine hemifumarate is dissolved in a dipolar aprotic solvent at a dissolution temperature. Dipolar aprotic solvents can include dimethylformamide (DMF), dimethylsulfoxide (DMSO), 1-methyl-2-pyrrolidinone, and dimethylacetamide (DMAC). The dissolution temperature can be 50°C or more. Preferably, the dissolution temperature is about 80°C. The solution is then combined with a halogenated hydrocarbon. The solution is then cooled, preferably to a temperature of about 30°C or less, and isolated.

Following treatment, the resulting solvate is collected (isolated) by suitable means as are known to skilled artisan and routiner alike, for example decanting, filtration (gravity or suction), or centrifugation, to mention just three. The collected polymorph or pseudopolymorph can be dried in air at room temperature or elevated temperature, or it can be dried in an oven at atmospheric or reduced pressure. However, care must be exercised during drying so as to not remove solvating solvent.

In one embodiment, the present invention provides a novel crystalline form of quetiapine hemifumarate, denominated form II, and its chloroform and methylene chloride solvates, and a method for making them.

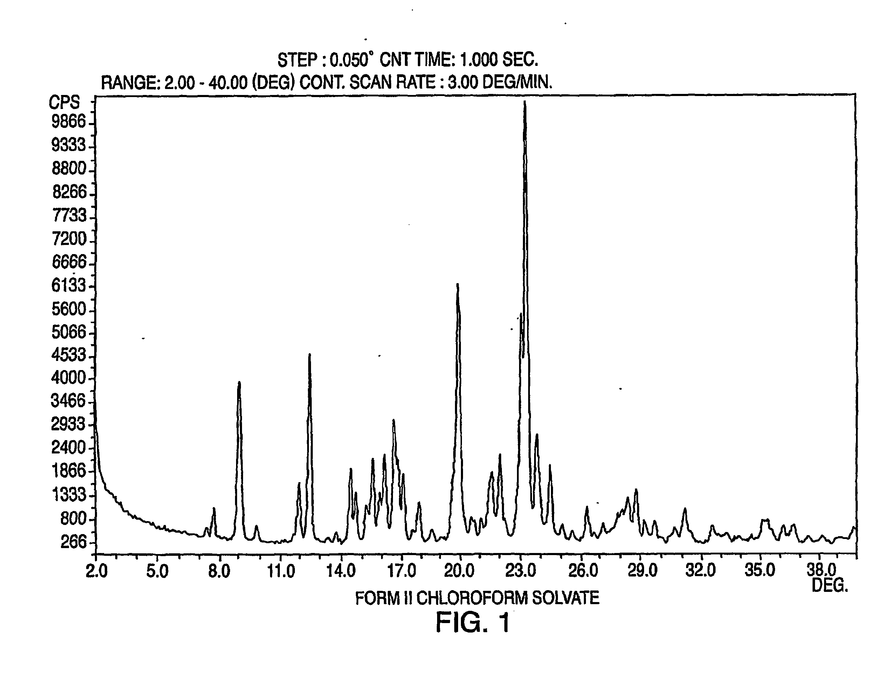

One characteristic of quetiapine hemifumarate form II and its halogenated hydrocarbon solvates is its powder x-ray diffraction pattern (XRD). Quetiapine hemifumarate form II is characterized by XRD reflections (peaks) at about 7.8°, 11.9°, 12.5°, 15.7°, 23.0°, and 23.4°, ± 0.2° 2θ. Quetiapine hemifumarate form II also exhibits x-ray reflections at 9.0°, 15.6°, 19.7°, 20.0°, 21.6°, and 23.8°, ± 0.2° 2θ. A typical x-ray diffraction diagram of quetiapine hemifumarate form II as its chloroform solvate is shown in Figure 1.

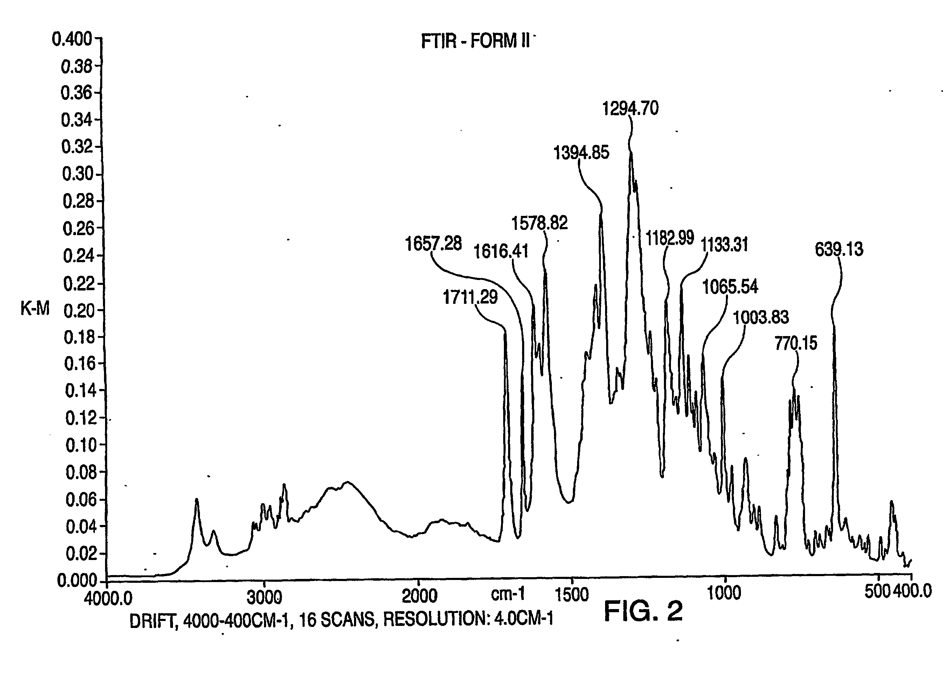

Another characteristic of quetiapine hemifumarate form II and its halogenated hydrocarbon solvates is its pattern of absorption bands in FTIR spectroscopy. Quetiapine hemifumarate form II is characterized by absorption bands at 639, 1112, 1395, 1616, 1711, and 3423 cm⁻¹. The FTIR spectrogram of quetiapine hemifumarate form II as its chloroform solvate is shown in Figure 2.

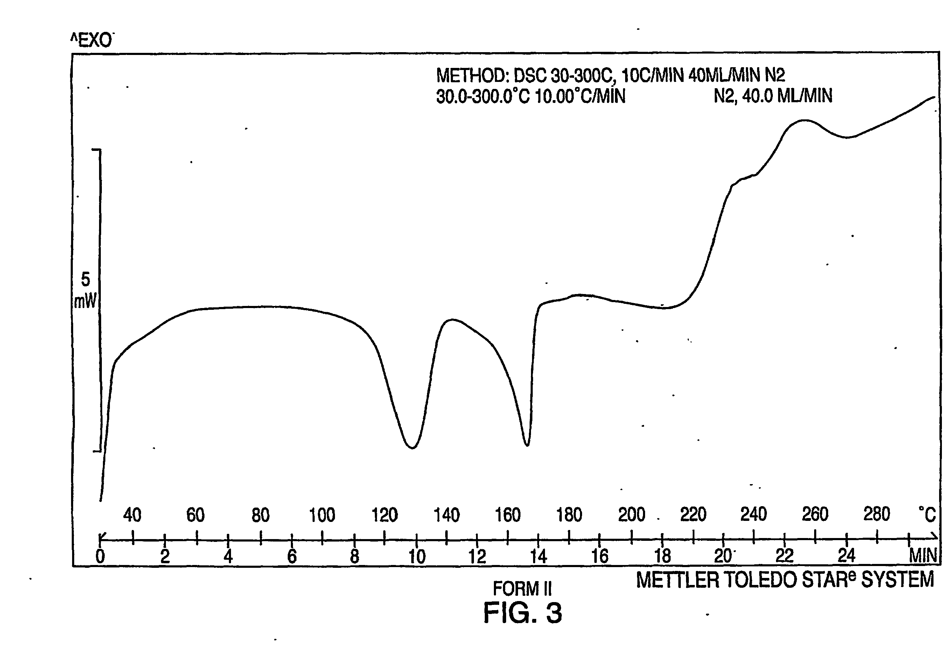

An additional characteristic of quetiapine hemifumarate form II and its halogenated hydrocarbon solvates is its thermogram in differential scanning calorimetry (DSC). The DSC thermogram of quetiapine hemifumarate form II as its chloroform solvate is shown in Figure 3. The DSC thermogram of quetiapine hemifumarate form II is characterized by endothermic peaks at about 130°C and at about 166°C.

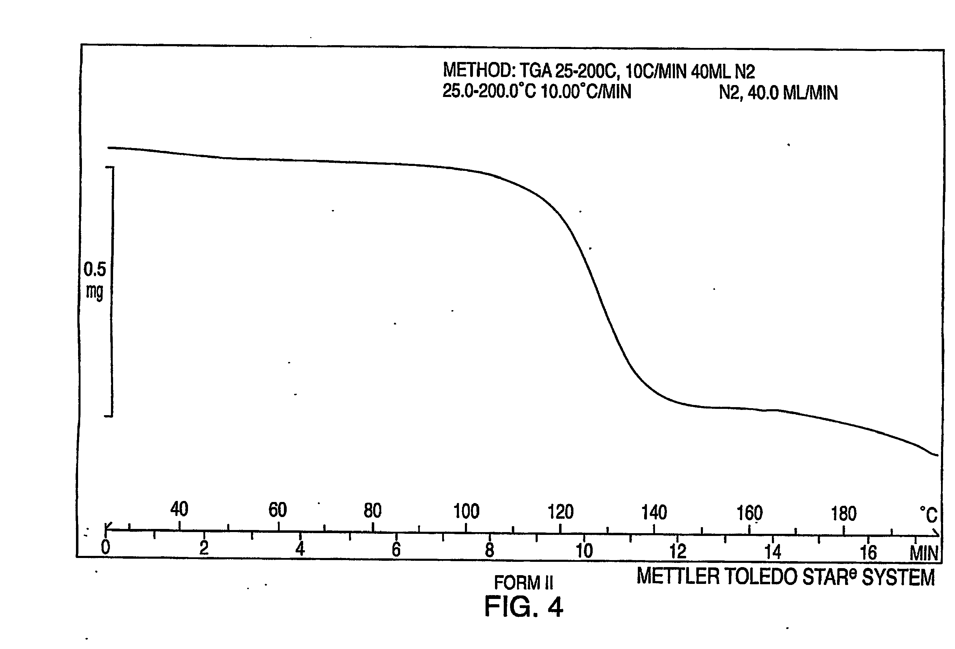

Quetiapine hemifumarate form II shows a loss-on-drying (LOD) of about 4.7% in TGA in the temperature range of between about 130°C and about 166°C. The TGA for quetiapine hemifumarate form II as its chloroform solvate in another embodiment of the present invention is shown in Figure 4.

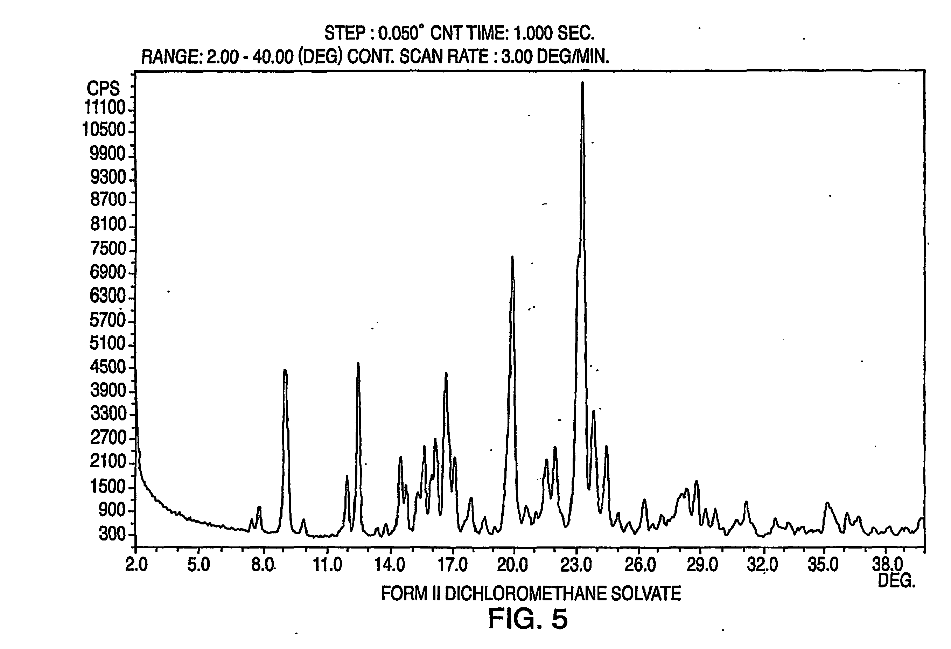

One characteristic of quetiapine hemifumarate form II dichloromethane solvate is its powder x-ray diffraction pattern (XRD). Quetiapine hemifumarate form II dichloromethane solvate is characterized by XRD reflections (peaks) at about 7.8°, 11.9°, 12.5°, 15.7°, 23.0°, and 23.4°, ± 0.2° 2θ. The x-ray diffraction diagram of quetiapine hemifumarate form II dichloromethane solvate is shown in Figure 5.

Another characteristic of quetiapine hemifumarate form II dichloromethane solvate is its absorption bands in FTIR at 639, 1112, 1395, 1616, 1711, and 3423 cm⁻¹.

In another embodiment, the present invention provides a reflux method for making a crystalline form of quetiapine hemifumarate having at least one characteristic of form II including the steps of: combining quetiapine hemifumarate and treating solvent, preferably methylene chloride or chloroform; refluxing the combination for a reflux time; cooling the combination after reflux; and isolating the crystalline quetiapine hemifumarate having at least one characteristic of form II.

The ratio of quetiapine hemifumarate to treating solvent is not critical. About 20 mL to 60 mL treating solvent per gram of quetiapine hemifumarate is generally sufficient. The reflux time is not critical. The skilled artisan will know to optimize the reflux time depending on, among other things, the quetiapine hemifumarate used as starting material and the ratio of quetiapine hemifumarate to treating solvent. Typically, reflux times of about 6 hours are sufficient. At the end of the reflux time, the combination is cooled, preferably to ambient temperature. The slurry can be and preferably is stirred for 10 to about 20 hours. Quetiapine hemifumarate having at least one characteristic of form II is then isolated by conventional techniques. In this and all reflux methods described herein, the recovering (isolating) can be by any means known in the art, for example filtration (gravity or suction) or centrifugation and decanting, to mention just two. Isolated solid is then preferably washed with an additional amount of treating solvent, and is preferably dried under vacuum from about 40° C to about 70° C overnight, more preferably at about 65° C.

In another embodiment, the present invention provides a solution method for making quetiapine hemifumarate having at least one characteristic of form II, and particularly chlorinated hydrocarbon solvates thereof, including the steps of: combining quetiapine hemifumarate and a treating solvent, preferably methylene chloride or chloroform, at a dissolution temperature, preferably 80° C or less, cooling the combination to a temperature of about 20° C or less, and isolating the crystalline quetiapine hemifumarate having at least one characteristic of form II.

When quetiapine hemifumarate form II as its chloroform solvate is desired, the reflux method is the preferred method, e.g., quetiapine hemifumarate is refluxed with chloroform for about 6 hours followed by cooling the slurry to ambient temperature and stirring for an additional time, preferably about 16 hours. The ratio of quetiapine hemifumarate to chloroform is not critical and can be between about 1% and about 10% (w/v). The solid is collected by filtration and dried overnight, preferably at a temperature of about 65°C (*see* Example 1). Quetiapine hemifumarate form II chloroform solvate samples prepared according to this embodiment of the invention typically exhibits XRD, FTIR and DSC patterns as seen in Figures 1, 2 and 3, respectively.

When quetiapine hemifumarate form II as its dichloromethane solvate is desired, either the solution method or the reflux method, including the steps of combining quetiapine hemifumarate with methylene chloride, refluxing, cooling and isolating the quetiapine hemifumarate form II product as its dichloromethane solvate, can be used.

Quetiapine hemifumarate form II as its dichloromethane solvate can made by the solution method, wherein quetiapine hemifumarate is dissolved in dimethylformamide, at a ratio of QTP:DMF of about 30% (w/v), at a dissolution temperature of about 50°C or more, preferably, about 80°C. The solution is added with methylene chloride [about 1:15 (v/v) QTP/DMF:methylene chloride], treated by cessation of heating and continued stirring overnight to permit formation of a precipitate. The precipitate is collected, preferably by filtration and dried for about 2 hours, preferably at a temperature of about 65°C (*see* Example 2).

In yet another embodiment, the present invention provides a novel crystalline form of quetiapine hemifumarate, denominated form III, and its chloroform and methylene chloride solvates, and a method for making them.

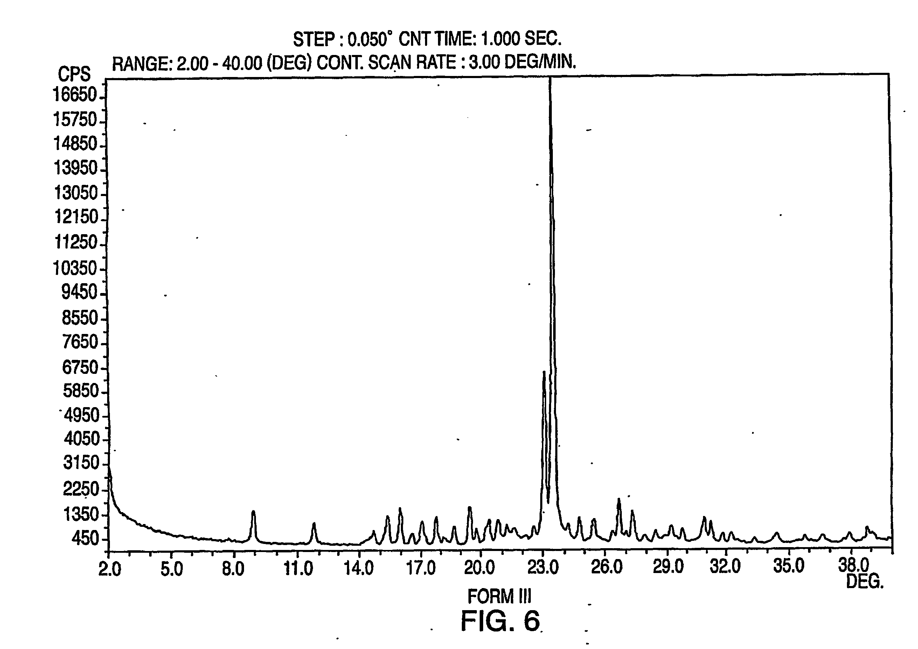

One characteristic of quetiapine hemifumarate form III, and its halogenated hydrocarbon solvates, is its powder x-ray diffraction pattern (XRD). Quetiapine hemifumarate form III chloroform solvate is characterized by XRD reflections (peaks) at about 8.9°, 11.8°, 15.3°, 19.4°, 23.0° and 23.4°, ± 0.2° 2θ. Quetiapine hemifumarate form III also exhibits x-ray reflections at 16.0°, 17.0°, 17.7°, 18.6°, 20.3°, 20.8°, 21.3°, 21.6°, 26.7°, and 27.4°, ± 0.2° 2θ. A typical x-ray diffraction diagram of quetiapine hemifumarate form III as its chloroform solvate is shown in Figure 6.

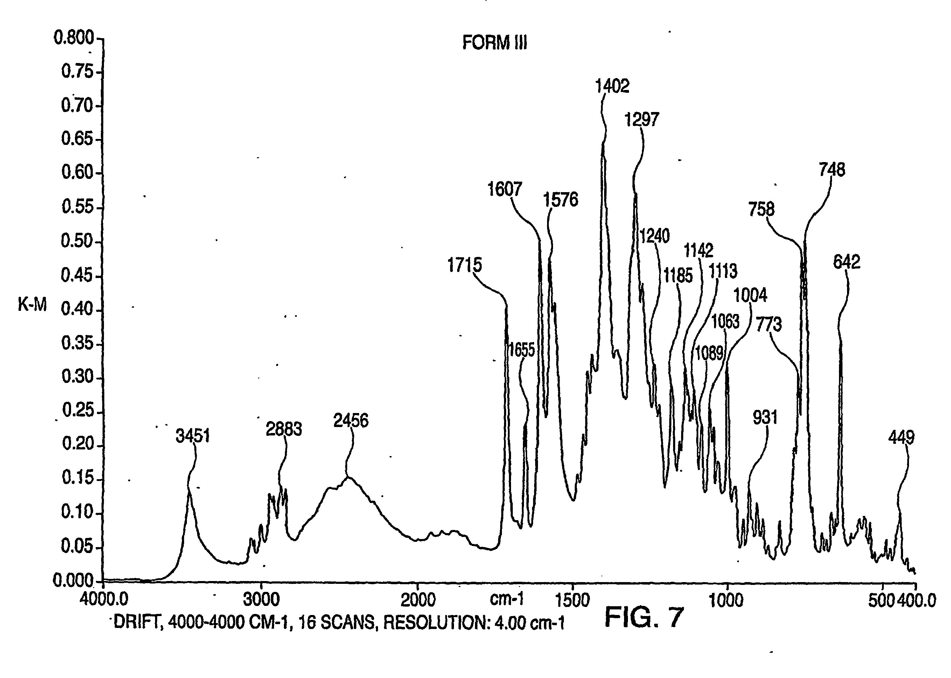

Another characteristic of quetiapine hemifumarate form III, and its halogenated hydrocarbon solvates, is its pattern of absorption bands in FTIR spectroscopy. Quetiapine hemifumarate form III is characterized by absorption bands at 748, 758, 1402, 1607, 1715, and 2883 cm⁻¹. The FTIR spectrum of quetiapine hemifumarate form III as its chloroform solvate is shown in Figure 7.

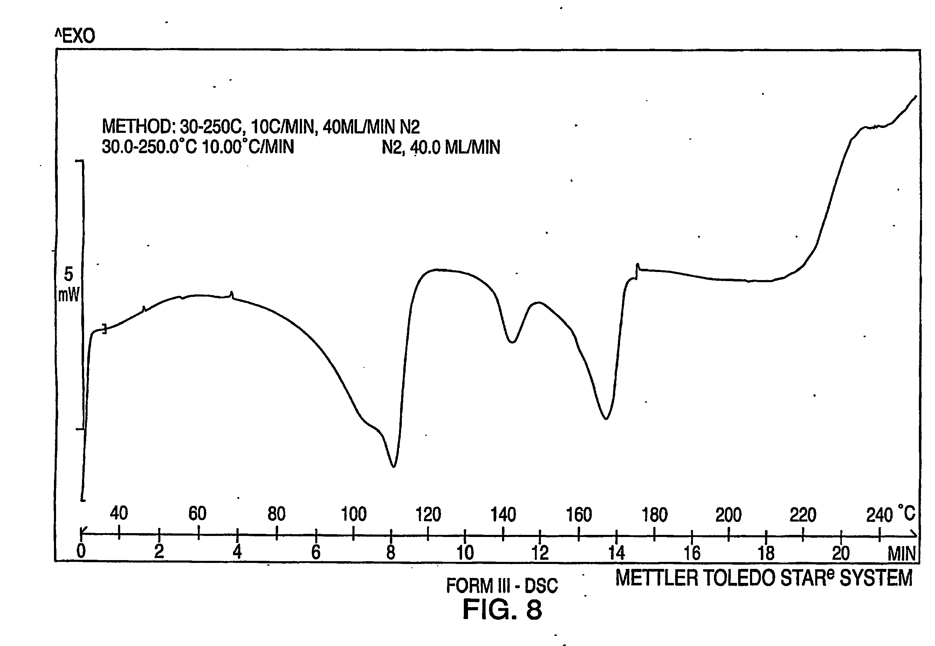

Another characteristic of quetiapine hemifumarate form III, and its halogenated hydrocarbon solvates, is its DSC thermogram, which exhibits endothermic peaks at about 111°C, about 142°C, and about 167°C. The DSC thermogram of quetiapine hemifumarate form III is shown in Figure 8. Thermogravimetric analysis (TGA) can also be applied to further characterize quetiapine hemifumarate form III as its chloroform solvate by a weight loss-on-drying (LOD) of between about 10% and about 19%, preferably between about 12% and about 13%, as shown by TGA.

In another embodiment, the present invention provides a solution method for making a crystalline form of quetiapine hemifumarate having at least one characteristic of form III, and particularly chlorinated hydrocarbon solvates thereof, including the steps of: combining quetiapine hemifumarate and a treating solvent, preferably a dipolar aprotic solvent, at a dissolution temperature, preferably, about 80°C or less, mixing the combination with chloroform, cooling the resulting mixture, and isolating the quetiapine hemifumarate having at least one characteristic of form III from the mixture.

The relative amount of treating solvent is not critical. Generally, between about 1 mL and about 2 mL of treating solvent are used are used for each gram of quetiapine hemifumarate to be treated. However, the routiner will know to adjust the proportions depending on, for example, the equipment to be used for treating. Quetiapine hemifumarate is dissolved in a dipolar aprotic solvent at a dissolution temperature. Dipolar aprotic solvents include dimethylformamide (DMF), dimethylsulfoxide (DMSO), 1-methyl-2-pyrrolidinone, and dimethylacetamide (DMAC). The dissolution temperature can be 50°C or more. Preferably, the dissolution temperature is about 80°C. The solution is then combined with a halogenated hydrocarbon, preferably chloroform. Generally, between about 10 mL and about 50 mL of chloroform are used for each gram of quetiapine hemifumarate. The solution is then cooled, preferably to a temperature of about 30°C or less, and isolated.

Similarly, the time of treatment is not critical but can vary from about 1 to about 48 hours, with 2 to 24 hours being typical.

Following treatment, the resulting solvate is collected (isolated) by suitable means as are known to skilled artisan and routiner alike, for example decanting, filtration (gravity or suction), or centrifugation, to mention just three. The collected quetiapine hemifumarate form III, and its halogenated hydrocarbon solvates, can be dried in air at room temperature or elevated temperature, or it can be dried in an oven at atmospheric or reduced pressure. However, care must be exercised during drying so as to not remove solvating solvent.

In another embodiment, the present invention provides a method of making quetiapine hemifumarate form III as its chloroform solvate. Quetiapine hemifumarate is dissolved in dimethylsulfoxide at a ratio of about 67% QTP:DMSO (w/v) at a dissolution temperature of about 50°C or more, preferably, about 80°C. The solution is added with dichloromethane [about 1:20 (v/v) QTP/DMSO:dichloromethane], treated by cessation of heating and continued stirring for 1 hour at ambient temperature. Formation of a precipitate occurs with cessation of stirring. After standing overnight, the precipitate is stirred, preferably for about 4 hours, collected, preferably by filtration and dried, preferably at a temperature of about 65°C (*see* Example 6).

In a still further embodiment, the present invention provides a method for making quetiapine hemifumarate form I, including the steps of providing a solution of quetiapine hemifumarate at a dissolution temperature in a dipolar aprotic solvent or an alkanol solvent, combining the solution with an anti-solvent to obtain a suspension, and isolating quetiapine hemifumarate form I from the suspension. The dissolution temperature is preferably about 80°C. Dipolar aprotic solvents useful in the practice of the present invention include dimethylformamide, dimethylsulfoxide, 1-methyl-2-pyrrolidinone, or dimethylacetamide. Anti-solvents useful in the practice of the present invention include ethylacetate, isopropylacetate, acetone, methyl *tert*-butyl ether (MTBE), or acetonitrile. Alkanol useful in the practice of the present invention includes isopropyl alcohol.

In yet another embodiment, the present invention provides a method for making quetiapine hemifumarate form I, including the steps of providing a solution of quetiapine hemifumarate at a dissolution temperature in a dipolar aprotic solvent or an alkanol solvent, cooling the solution to a temperature of about 30°C or less, and isolating quetiapine hemifumarate form I from the mixture. The dissolution temperature is preferably about 80°C. The dipolar aprotic solvent can contain water. A dipolar aprotic solvent useful in the practice of the present invention includes dimethylformamide. Alkanol useful in the practice of the present invention includes isopropyl alcohol.

In another embodiment, the present invention provides post-suspension and post-crystallization treatment methods for crystalline forms of quetiapine hemifumarate, preferably form I made by any of the embodiments of the method of the present invention.

The post-suspension method includes the steps of combining the isolated quetiapine hemifumarate form I with a post-suspending solvent selected from dialkyl ketones, aromatic hydrocarbons, cyanoalkanes, dialkyl ethers, and methylene chloride; refluxing the combination for a reflux time; cooling the combination to ambient temperature; optionally agitating the suspension for an agitating time; and isolating quetiapine hemifumarate form I.

Dialkyl ketones have the general formula R₁C(O)R₂, where R₁ and R₂ are independently a linear or branched alkyl group having up to 4 carbon atoms. Aromatic hydrocarbons are exemplified by benzene, toluene, and the tertalins. Cyanoalkanes have the general formula RCN, where R is a linear or branched alkyl group having up to 6 carbon atoms. Dialkyl ethers have the general formula R₁-O-R₂, where R₁ and R₂ are independently a linear or branched alkyl group having up to 4 carbon atoms. Examples of post-suspension solvents include acetone, toluene, acetonitrile, dichloromethane, and methyl *t*-butyl ether. Reflux times are generally between about 1 and about 6 hours. When an agitation time is used, it is not critical.

The post-crystallization method includes the steps of: a) refluxing a solution of the isolated quetiapine hemifumarate form I in a post-crystallization solvent selected from lower alkanols, cyclic ethers, ethyl acetate, and water for a reflux time, cooling the solution to ambient temperature whereby a suspension is formed; optionally agitating the suspension for an agitation time; and isolating the quetiapine hemifumarate form I.

The cyclic ethers are exemplified by tetrahydrofuran (THF) and the dioxanes. The reflux time in the post-crystallization method is not critical and can be 1 to about 10 hours. When an agitation time is used, it is not critical.

In yet another embodiment, the present invention provides a pharmaceutical composition including one or more of quetiapine hemifumarate form II chloroform solvate, form II dichloromethane solvate, or form III chloroform solvate. The pharmaceutical composition can be in the form of a solid oral dosage form (e.g., compressed tablets or capsules), or it can be in the form of a liquid oral dosage form, e.g., a solution or oral suspension.

In one aspect, the present invention relates to micronized quetiapine hemifumarate including a plurality of quetiapine hemifumarate particles wherein the mean particle size (d_{.05}) is about 2 µm to about 7 µm and 10 volume percent or less of the plurality of particles have a particle diameter equal to or greater than about 30 µm, preferably 20 µm.

In another aspect, the present invention relates to micronized quetiapine hemifumarate including a plurality of quetiapine hemifumarate particles obtained by comminution using a fluid energy mill, wherein the mean particle size (d_{.05}) is about 2 µm to about 7 µm and 10 volume percent or less of the plurality of particles have a particle diameter equal to or greater than about 10 µm.

A fluid energy mill, or "micronizer", is an especially preferred type of mill for its ability to produce particles of small size in a narrow size distribution, i.e., micronized material. As those skilled in the art are aware, micronizers use the kinetic energy of collision between particles suspended in a rapidly moving fluid (typically air) stream to cleave the particles. An air jet mill is a preferred fluid energy mill. The suspended particles are injected under pressure into a recirculating particle stream. Smaller particles are carried aloft inside the mill and swept into a vent connected to a particle size classifier such as a cyclone. The feedstock should first be milled to about 150 to 850 µm which may be done using a conventional ball, roller, or hammer mill.

The starting material may have an average particle size of about 20-100 microns.

The material is fed into the micronization system in a controlled feed rate by means of a screw feeder or a vibratory feeder. The air jet mill is operated with controlled air pressures. For the Microgrinding MC-500 KX, the feed rate is 40-80 kg/hr, the Feed air pressure is 6-8.5 bar and the grinding air is 3-6 bar.

Micronizationization can also be accomplished with a pin mill. The starting material may have an average particle size of about 20-100 microns. The material is fed into the mill system in a controlled feed rate by means of a screw feeder or a vibratory feeder. The mill is operated with controlled speed. For the Alpine UPZ 160, the feed rate is 60-75 kg/hr, the mill speed is 7,000-15,000 rpm.

Compressed tablets can be made by dry or wet granulation methods as is known in the art. In addition to the pharmaceutically active agent or drug, compressed tablets contain a number of pharmacologically inert ingredients, referred to as excipients. Some excipients allow or facilitate the processing of the drug into tablet dosage forms. Other excipients contribute to proper delivery of the drug by, for example, facilitating disintegration.

Excipients can be broadly classified according to their intended function. However, it must be kept in mind that a particular excipient can be capable of acting in more than one way.

Diluents increase the bulk of a solid pharmaceutical composition and may make a pharmaceutical dosage form containing the composition easier for the patient and caregiver to handle. Diluents for solid compositions include, for example, microcrystalline cellulose (e.g., AVICEL^{®}, microfine cellulose, lactose, starch, pregelitinized starch, calcium carbonate, calcium sulfate, sugar, dextrates, dextrin, dextrose, dibasic calcium phosphate dihydrate, tribasic calcium phosphate, kaolin, magnesium carbonate, magnesium oxide, maltodextrin, mannitol, polymethacrylates (e.g., EUDRAGIT^{®}), potassium chloride, powdered cellulose, sodium chloride, sorbitol and talc.

Solid pharmaceutical compositions that are compacted into a dosage form like a tablet may include excipients whose functions include helping to bind the active ingredient and other excipients together after compression. Binders for solid pharmaceutical compositions include acacia, alginic acid, carbomer (e.g., carbopol), carboxymethylcellulose sodium, dextrin, ethyl cellulose, gelatin, guar gum, hydrogenated vegetable oil, hydroxyethyl cellulose, hydroxypropyl cellulose (e.g., KLUCEL^{®}), hydroxypropyl methyl cellulose (e.g., METHOCEL^{®}), liquid glucose, magnesium aluminum silicate, maltodextrin, methylcellulose, polymethacrylates, povidone (e.g., KOLLIDON^{®}, PLASDONE^{®}), pregelatinized starch, sodium alginate and starch. The dissolution rate of a compacted solid pharmaceutical composition in the patient's stomach may be increased by the addition of a disintegrant to the composition.

Disintegrants include alginic acid, carboxymethylcellulose calcium, carboxymethylcellulose sodium (e.g., AC-DI-SOL^{®}, PRIMELLOSE^{®}), colloidal silicon dioxide, croscarmellose sodium, crospovidone (e.g., KOLLIDON^{®}, POLYPLASDONE^{®}), guar gum, magnesium aluminum silicate, methyl cellulose, microcrystalline cellulose, polacrilin potassium, powdered cellulose, pregelatinized starch, sodium alginate, sodium starch glycolate (e.g., EXPLOTAB^{®}) and starch.

Glidants can be added to improve the flow properties of non-compacted solid compositions and improve the accuracy of dosing. Excipients that may function as glidants include colloidal silicon dixoide, magnesium trisilicate, powdered cellulose, starch, talc and tribasic calcium phosphate.

When a dosage form such as a tablet is made by compaction of a powdered composition, the composition is subjected to pressure from a punch and die. Some excipients and active ingredients have a tendency to adhere to the surfaces of the punch and die, which can cause the product to have pitting and other surface irregularities. A lubricant can be added to the composition to reduce adhesion and ease release of the product from the die. Lubricants include magnesium stearate, calcium stearate, glyceryl monostearate, glyceryl palmitostearate, hydrogenated castor oil, hydrogenated vegetable oil, mineral oil, polyethylene glycol, sodium benzoate, sodium lauryl sulfate, sodium stearyl fumarate, stearic acid, talc and zinc stearate.

Flavoring agents and flavor enhancers make the dosage form more palatable to the patient. Common flavoring agents and flavor enhancers for pharmaceutical products that may be included in the composition of the present invention include maltol, vanillin, ethyl vanillin, menthol, citric acid, fumaric acid ethyl maltol, and tartaric acid.

Compositions may also be colored using any pharmaceutically acceptable colorant to improve their appearance and/or facilitate patient identification of the product and unit dosage level.

Of course, wet or dry granulate can also be used to fill capsules, for example gelatin capsules. The excipients chosen for granulation when a capsule is the intended dosage form may or may not be the same as those used when a compressed tablet dosage form is contemplated.

Selection of excipients and the amounts to use may be readily determined by the formulation scientist based upon experience and consideration of standard procedures and reference works in the field.

The present invention is further described by the following nonlimiting examples.

### EXAMPLES

### Quetiapine Hemifumarate Form II Chlorform Solvate

### Example 1A:

Quetiapine hemifumarate (2 g) is slurried in chloroform (80 mL) and refluxed for 6 hours. The slurry is then cooled to ambient temperature and then stirred for about 16 hours. The solid is then collected by filtration and dried 24 hrs. in a vacuum oven at 65°C to yield 1.15 g of a solid. The solid has the XRD, FTIR, and DSC shown in Figures 1, 2, and 3, respectively.

### Example 1B:

Quetiapine hemifumarate (2 g) is slurried in chloroform (65 mL) and refluxed for 6 hours. The slurry is then cooled to ambient temperature and then stirred for about 16 hours. The solid is then collected by filtration and dried 24 hrs. in a vacuum oven at 65°C to yield 1.15 g of a solid. The solid has the XRD, FTIR, and DSC shown in Figures 1, 2, and 3, respectively.

### Quetiapine Hemifumarate Form II Dichloromethane Solvate

### Example 2:

Quetiapine hemifumarate (4 g) is dissolved in dimethylformamide (13 mL) with heating at 80°C, followed by addition of methylene chloride (250 mL), resulting in a clear mixture. Heating is discontinued and the mixture is stirred overnight, during which time a precipitate forms. The precipitate is collected by filtration and dried for 2 hours at 65°C.

### Example 3:

Quetiapine hemifumarate (4 g) is dissolved in dimethylsulfoxide (7 mL) with heating at 80°C, followed by addition of dichloromethane (200 mL) to form a clear solution. Heating is discontinued and the solution is allowed to stir about 2 days, resulting in a yellowish mixture. The mixture is filtered and the solids are collected and dried.

### Example 4:

Quetiapine hemifumarate (4 g) is dissolved in 1-methyl-2-pyrrolidinone (8 mL) with heating at 80°C, followed by addition of dichloromethane (200 mL) to form a clear solution. Heating is discontinued and the solution is allowed to stir overnight at room temperature during which time a precipitate forms. The mixture is allowed to stand at room temperature for 2 days, following which time the precipitate is collected by filtration and dried.

### Example 5:

Quetiapine hemifumarate (4 g) is dissolved in dimethylacetamide (7 mL) and dichloromethane (200 mL) is added, resulting in a clear solution. Heating is discontinued and the mixture is allowed to stir for 2 hours at room temperature. The mixture is filtered and the solids are collected and dried for 2 hours at 65°C.

### Quetiapine Hemifumarate Form III Chloroform Solvate

### Example 6:

Quetiapine hemifumarate (4 g) is dissolved in dimethylsulfoxide (6 mL) with heating to 80°C, followed by addition of dichloromethane (200 mL) to form a clear solution. The heating is discontinued and the solution is stirred for 1 hour at room temperature. Chloroform (70 mL) is then added and the resulting mixture is stirred overnight. The stirring is discontinued and the mixture is allowed to stand for another night. After formation of a precipitate, the mixture is stirred for 4 hours and then filtered to isolate the precipitate. The precipitate is dried at 65° C.

### Example 7:

Quetiapine hemifumarate (4 g) is partially dissolved in dimethylsulfoxide (4 mL) at 80°C. Chloroform (50 mL) is added and solids formed. Additional chloroform (150 mL) is added and the solids are collected by filtration.

### Example 8:

Quetiapine hemifumarate (4g) is dissolved in 1-methyl-2-pyrrolidinone (8 mL) with heating at 80°C, followed by addition of chloroform (200 mL). The mixture is stirred at room temperature for 2 days and filtered to collect the precipitate formed.

### Example 9:

Quetiapine hemifumarate (4 g) is dissolved in dimethylacetamide (7 mL) with heating at 80°C, followed by addition of chloroform (200 mL). Heating is discontinued and the mixture is allowed to stir at room temperature for about 2 days. The mixture is further cooled and filtered to collect the precipitate which is dried for 2 hours at 65°C.

### Quetiapine Hemifumarate Form I

### Example 10:

The following general procedure was repeated in the examples reported below. The desired amount of quetiapine hemifumarate was dissolved in the desired solvent (e.g., water, alkanol, and dipolar aprotic solvents) at a dissolution temperature (nominally 80°C) and the solution was combined with thye desiored antisolvent, whereby for I was obtained. The results are summarized in the table below.

**Table 10A**

| **Sample** | **Description** |
|---|---|
| 10 A | Dissolved in IPA, 38 mL/g at 80°C, cooled, filtered and dried at 65°C |
| 10 B | Dissolved in DMF, 3.25 mL/g at 80°C, precipitated with isopropylacetate, 14mL/g, filtered and dried at 65°C |
| 10 C | Dissolved in DMF, 3.25 mL/g at 80°C, precipitated with acetone, 65mL/g, filtered |
| 10 D | Dissolved in DMF, 3.25 mL/g at 80°C, precipitated with acetone, 65mL/g, filtered and dried at 65°C |
| 10 E | Dissolved in DMF, 2.50 mL/g at 80°C, precipitated with acetonitrile, 6.75mL/g, filtered and dried at 65°C |
| 10 F | Dissolved in DMF, 2.50 mL/g at 80°C, precipitated with toluene, 50mL/g, filtered |
| 10 G | Dissolved in DMSO, 1.75 mL/g at 80°C, precipitated with water, 8.75mL/g, filtered and dried at 65°C |
| 10 H | Dissolved in DMSO, 1.75 mL/g at 80°C, precipitated with ethylacetate, 50mL/g, filtered and dried at 65°C |
| 10 I | Dissolved in IPA, 37.5 mL/g at 80°C, precipitated with ethylacetate, 62mL/g, filtered |
| 10 J | Dissolved in IPA, 37.5 mL/g at 80°C, precipitated with isopropylacetate, 75mL/g, filtered |
| 10 K | Dissolved in IPA, 37.5 mL/g at 80°C, precipitated with acetone, 75mL/g, filtered |
| 10 L | Dissolved in IPA, 37.5 mL/g at 80°C, precipitated with MTBE, 75mL/g, filtered |
| 10 M | Dissolved in methanol, 22.5 mL/g at 80°C, precipitated with isopropylacetate, 75mL/g, filtered |
| 10 N | Dissolved in DMSO, 1.75 mL/g at 80°C, precipitated with dichloromethane, 50mL/g, filtered and dried at 65°C |
| 10 O | Dissolved in DMSO, 1.75 mL/g at 80°C, precipitated with toluene, 50mL/g, filtered and dried at 65°C |
| 10 P | Dissolved in DMF, 2.50 mL/g at 80°C, precipitated with MTBE, 7.25mL/g, filtered |
| 10 Q | Dissolved in DMF, 2.50 mL/g at 80°C, precipitated with MTBE, 7.25mL/g, filtered and dried at 65°C |
| 10 R | Dissolved in DMF, 2.50 mL/g at 80°C, precipitated with toluene, 50mL/g, filtered and dried at 65°C |
| 10 S | Dissolved in DMSO, 1.75 mL/g at 80°C, precipitated with acetone, 50mL/g, filtered |
| 10 T | Dissolved in DMSO, 1.75 mL/g at 80°C, precipitated with acetonitrile, 8.75 mL/g, filtered |
| 10 U | Dissolved in DMSO, 1.75 mL/g at 80°C, precipitated with isobutanol, 50 mL/g, filtered and dried at 65°C |
| 10 V | Dissolved in DMF, 3.25 mL/g at 80°C, precipitated with ethylacetate, 25 mL/g, filtered |
| 10 W | Dissolved in DMF, 3.25 mL/g at 80°C, precipitated with ethylacetate, 25 mL/g, filtered and dried at 65°C |
| 10 X | Dissolved in DMF, 2.5 mL/g at 80°C, precipitated with isobutanol, 50mL/g, filtered |
| 10 Y | Dissolved in DMF, 2.5 mL/g at 80°C, precipitated with isobtanol, 50mL/g, filtered and dried at 65°C |
| 10 Z | Dissolved in DMSO, 1.75 mL/g at 80°C, precipitated with ethylacetate, 50 mL/g, filtered |
| 10 AA | Dissolved in DMF, 2.5 mL/g at 80°C, precipitated with water, 25mL/g, filtered |
| 10 BB | Dissolved in DMF, 2.5 mL/g at 80°C, precipitated with water, 25mL/g, filtered and dried at 65°C |
| 10 CC | Dissolved in 1-methyl-2-pyrrolidone, 2 mL/g at 80°C, precipitated with acetone, 50mL/g, filtered and dried at 65°C |
| 10 DD | Dissolved in 1-methyl-2-pyrrolidone, 2 mL/g at 80°C, precipitated with isobutanol, 10.5mL/g, filtered and dried at 65°C |
| 10 EE | Dissolved in dimethylacetamide, 1.75 mL/g at 80°C, precipitated with water, 50mL/g, filtered |
| 10 FF | Dissolved in dimethylacetamide, 1.75 mL/g at 80°C, precipitated with ethylacetate, 12.5mL/g, filtered |
| 10 GG | Dissolved in dimethylacetamide, 1.75 mL/g at 80°C, precipitated with isopropylacetate, 9.5mL/g, filtered |
| 10 HH | Dissolved in dimethylacetamide, 1.75 mL/g at 80°C, precipitated with isopropylacetate, 9.5mL/g, filtered and dried at 65°C |
| 10 II | Dissolved in dimethylacetamide, 1.75 mL/g at 80°C, precipitated with acetonitrile, 6.25mL/g, filtered |
| 10 JJ | Dissolved in dimethylacetamide, 1.75 mL/g at 80°C, precipitated with MTBE, 8.75mL/g, filtered |
| 10 KK | Dissolved in dimethylacetamide, 1.75 mL/g at 80°C, precipitated with acetone, 12.5mL/g, filtered and dried at 65°C |
| 10 LL | Dissolved in dimethylacetamide, 1.75 mL/g at 80°C, precipitated with acetonitrile, 6.25mL/g, filtered and dried at 65°C |
| 10 MM | Dissolved in dimethylacetamide, 1.75 mL/g at 80°C, precipitated with MTBE, 8.75mL/g, filtered and dried at 65°C |
| 10 NN | Dissolved in 1-methyl-2-pyrrolidone, 2 mL/g at 80°C, precipitated with ethylacetate, 50mL/g, filtered |
| 10 OO | Dissolved in 1-methyl-2-pyrrolidone, 2 mL/g at 80°C, precipitated with acetonitile, 12.5mL/g, filtered and dried at 65°C |
| 10 PP | Dissolved in dimethylacetamide, 1.75 mL/g at 80°C, precipitated with acetone, 12.5mL/g, filtered |
| 10 QQ | Dissolved in 1-methyl-2-pyrrolidone, 2 mL/g at 80°C, precipitated with water, 50mL/g, filtered |
| 10 RR | Dissolved in 1-methyl-2-pyrrolidone, 2 mL/g at 80°C, precipitated with ethylacetate, 50mL/g, filtered and dried at 65°C |
| 10 SS | Dissolved in 1-methyl-2-pyrrolidone, 2 mL/g at 80°C, precipitated with MTBE, 37.5mL/g, filtered |
| 10 TT | Dissolved in 1-methyl-2-pyrrolidone, 2 mL/g at 80°C, precipitated with MTBE, 37.5mL/g, filtered and dried at 65°C |
| 10 UU | Dissolved in DMF, 2.5 mL/g at 80°C, precipitated with acetonitrile, 6.75 mL/g, filtered |
| 10 VV | Dissolved in IPA, 38 mL/g at 80°C, cooled, filtered |
| 10 WW | Dissolved in DMF, 3.25 mL/g at 80°C, precipitated with isopropylacetate, 14 mL/g, filtered |
| 10 XX | Dissolved in DMSO, 1.75 mL/g at 80°C, precipitated with water, 8.75mL/g, filtered |
| 10 AAA | Dissolved in DMSO, 1.75 mL/g at 80°C, precipitated with isopropylacetate, 50mL/g, filtered and dried at 65°C |
| 10 BBB | Dissolved in water (25 mL/g) and DMF (3.25mL/g), at 80°C, cooled and filtered |
| 10 CCC | Dissolved in 1-methyl-2-pyrrolidone, 2 mL/g at 80°C, precipitated with water, 50mL/g, filtered and dried at 65°C |
| 10 DDD | Dissolved in dimethylacetamide, 1.75 mL/g at 80°C, precipitated with ethylacetate, 12.5mL/g, filtered and dried at 65°C |
| 10 EEE | Dissolved in 1-methyl-2-pyrrolidone, 2 mL/g at 80°C, precipitated with isopropylacetate, 12.5mL/g, filtered |
| 10 FFF | Dissolved in 1-methyl-2-pyrrolidone, 2 mL/g at 80°C, precipitated with isopropylacetate, 12.5mL/g, filtered and dried at 65°C |
| 10 GGG | Dissolved in 1-methyl-2-pyrrolidone, 2 mL/g at 80°C, precipitated with acetone, 50mL/g, filtered |
| 10 HHH | Dissolved in 1-methyl-2-pyrrolidone, 2 mL/g at 80°C, precipitated with acetonitrile, 12.5mL/g, filtered |
| 10 III | Dissolved in 1-methyl-2-pyrrolidone, 2 mL/g at 80°C, precipitated with isobutanol, 10.5mL/g, filtered |
| 10 JJJ | Dissolved in dimethylacetamide, 1.75 mL/g at 80°C, precipitated with water, 50mL/g, filtered and dried at 65°C |
| 10 KKK | Dissolved in DMSO, 1.75 mL/g at 80°C, precipitated with isopropylacetate, 50mL/g, filtered |
| 10 LLL | Dissolved in DMSO, 1.75 mL/g at 80°C, precipitated with acetone, 50mL/g, filtered and dried at 65°C |
| 10 MMM | Dissolved in DMSO, 1.75 mL/g at 80°C, precipitated with acetonitrile, 8.75 mL/g, filtered and dried at 65°C |
| 10 NNN | Dissolved in DMSO, 1.75 mL/g at 80°C, precipitated with MTBE, 37.5mL/g, filtered |
| 10 OOO | Dissolved in DMSO, 1.75 mL/g at 80°C, precipitated with MTBE, 37.5mL/g, filtered and dried at 65°C |
| 10 PPP | Dissolved in DMSO, 1.75 mL/g at 80°C, precipitated with toluene, 50mL/g, filtered |
| 10 QQQ | Dissolved in DMSO, 1.75 mL/g at 80°C, precipitated with isobutanol, 50mL/g, filtered |
| 10 RRR | Dissolved in water (25 mL/g) and DMF (3.25mL/g), at 80°C, cooled and filtered and dried at 65°C |
| 10 SSS | Dissolved in IPA, 37.5 mL/g at 80°C, precipitated with ethylacetate, 62mL/g, filtered and dried at 65°C |
| 10 TTT | Dissolved in IPA, 37.5 mL/g at 80°C, precipitated with isopropylacetate, 75mL/g, filtered and dried at 65°C |
| 10 UUU | Dissolved in IPA, 37.5 mL/g at 80°C, precipitated with acetone, 75mL/g, filtered and dried at 65°C |
| 10 VVV | Dissolved in IPA, 37.5 mL/g at 80°C, precipitated with acetonitrile, 87.5mL/g, filtered |
| 10 WWW | Dissolved in methanol, 22.5 mL/g at 80°C, precipitated with ethylacetate, 75mL/g, filtered |
| 10 XXX | Dissolved in methanol, 22.5 mL/g at 80°C, precipitated with ethylacetate, 75mL/g, filtered and dried at 65°C |
| 10 YYY | Dissolved in methanol, 22.5 mL/g at 80°C, precipitated with acetone, 75mL/g, filtered |
| 10 ZZZ | Dissolved in methanol, 22.5 mL/g at 80°C, precipitated with acetone, 75mL/g, filtered and dried at 65°C |
| 10 NW | Dissolved in IPA, 37.5 mL/g at 80°C, precipitated with acetonitrile, 87.5mL/g, filtered and dried at 65°C |

### Treatment of Quetiapine Hemifumarate

### Example 11:

The desired quantity of QTP was combined with the desired number of volumes of solvent (1 volume = 1g/mL). The resulting combination was heated to reflux, whereby at least partial dissolution occurred. The resulting mixture was cooled to a crystallization temperature, typically room temperature, and stirred for a holding time. The crystalls were then recovered in the usual way. The results are given in the table below.

**Table 11A**

| Exp. No | Experimental conditions Starting material: QTP.hemifumarate | Yield | Polymorph |
|---|---|---|---|
| LB-56 | acetone (20 vol.),slurry at reflux for 6hrs.and then stirring at R.T. for additional 17 hrs. Filtration; washing with acetone( 2*10ml) and drying in vaccum oven 65°C/22.5hrs. | 93% | a similar crystal form as starting material. |
| LB-57 | Toluene (60 vol.),slurry at reflux for 13hrs. → partially dissolution → stirring at R.T. for 2hrs. →Cooling at 4°C during 16.5hrs. Filtration; washing with toluene (2*10ml) and drying in vaccum oven 65°C/24hrs. | 37% | a similar crystal form as starting material with additional peaks at 9.7.11.5.12.4,13.9.16.7. 23.5,28.7 |
| LB-58 | acetonitrile (45 vol.),slurry at reflux for 6hrs →partially dissolution → stirring at R.T. for 18.5hrs. Filtration; washing with acetonitrile(2*10ml) and drying in vaccum oven 65°C/24hrs. | 94.5% | a similar crystal form as starting material. |
| LB-59 | water (15 vol.), reflux for 20 minutes → dissolution → stirring at R.T. for 4hrs. Filtration; washing with water( 2*10ml) and drying in vaccum oven 65°C/20hrs. | 87% | a similar crystal form as starting material. |
| LB-60 | 1-Butanol (19 vol.), reflux for 20 minutes → dissolution **→** stirring at R.T. for 3hrs. Filtration; washing with 1-Butanol (2*10ml) and drying in vaccum oven 65°C/18hrs. | 94.5% | a similar crystal form as starting material. |
| LB-62 | MTBE (35 vol.), slurry at reflux for 6hrs.and then stirring at R.T. for additional 16hrs. Filtration; washing with MTBE (2*10ml) and drying in vaccum oven 65°C/24hrs. | 98.5% | a similar crystal form as starting material. |
| LB- 64 | IPA (25 vol.), reflux for 45 minutes → dissolution → stirring at R.T. for 1.25hrs. Filtration; washing with IPA (2*10ml) and drying in vaccum oven 65°C/19.5hrs. | 91% | a similar crystal form as starting material. |
| LB-65 | 1,4-dioxane (25 vol.), reflux for 1/2hr **→** dissolution **→** cooling to R.T. and then in an lce-bath for 1/2hr → the solution was stirred for additional 4hrs.at R.T. Filtration; washing with 1,4-dioxane (2*10ml) and drying in vaccume oven 65°C/15hrs. | 48% | a similar crystal form as startying material. |
| LB-66 | MEK (40 vol.), reflux for 6hrs. **→** dissolution → stirring at R.T. for 15hrs. Filtration; washing with MEK (2*10ml) and drying in vaccuv oven 65°C/24hrs. | 86.5% | a similar crystal form as starting material. |
| LB-67 | 1 -Propanol (15 vol.), reflux for 1/2hr. → dissolution → stirring at R.T. for 2.5 hrs. Filtration; washing with 1-Propanol (2*10ml) and drying in vaccum oven 65°C/15.5hrs. | 89.5% | a similar crystal form as starting material. |
| LB-68 | 2-Butanol (25 vol.), reflux for 45 minutes → dissolution → stirring at R.T. for 4hrs. Filtration; washing with 2-Butanol (2*10ml) and drying in vaccum oven 65°C/24hrs. | 90% | a similar crystal form as starting material. |
| LB-69 | ethyl-acetate (60 vol.), reflux for 7.5hrs. **→** partially dissolution → stirring at R.T. for 63hrs. Filtration; washing with ethyl-acetate (2*10ml) and drying In vaccum oven 65°C/22.5hrs. | 69% | a similar crystal form as starting material. |
| | ** Evaporation of the mother-liquid gave the same crystal form as starting materiai.(LB-69-1) | | |
| LB-70 | abs. EtOH (15 vol.), reflux for 1 hr. → dissolution → stirring at R.T. for 3.5hrs. Filtration; washing with abs. EtOH (2*10ml)and drying in vaccum oven 65°C/16hrs. | 86.5% | a similar crystal form as startying material. |
| LB-71 | THF (20 vol.), reflux for 1hr. → dissolution → stirring at R.T. for 3hrs. Filtration; washing with THF (2*10ml) and drying in vaccum oven 65°C/15hrs. | 54% | a similar crystal form as starting material. |
| | **Evaporation of the mother-liquid gave the same crystal form as starting material.(LB-71-1) | | |
| LB-72 | MeOH (22.5 vol.), reflux for 1hr. → dissolution → stirring at R.T. for 1.5hr. Filtration; washing with MeOH (2*10ml) and drying in vaccum oven 65°C/15hrs. | 48% | a similar crystal form as starting material. |

Further aspects and features of the present invention are set out in the following numbered clauses:
1. A crystalline form of quetiapine hemifumarate having at least one characteristic selected from the group consisting of:
   1) x-ray reflections at 7.8°, 11.9°, 12.5°, 15.7°, 23.0°, and 23.4°, ± 0.2° 2θ,
   2) absorption bands in FTIR spectroscopy at 639, 1112, 1395, 1616, 1711, and 3423 cm⁻¹ and
   3) a differential scanning calorimetric thermogram with endothermic peaks at about 130°C and at about 166°C.
2. The crystalline form of quetiapine hemifumarate of clause 1 characterized by x-ray reflections at 7.8°, 11.9°, 12.5°, 15.7°, 23.0°, and 23.4°, ± 0.2° 2θ.
3. The crystalline form of quetiapine hemifumarate of clause 2 further characterized by x-ray reflections at 9.0°, 15.6°, 19.7°, 20.0°, 21.6°, and 23.8°, ± 0.2° 2θ.
4. The crystalline quetiapine hemifumarate of clause 3 having an x-ray diffraction diagram substantially as shown in Figure 1.
5. The crystalline quetiapine hemifumarate of clause 1 having absorption bands in FTIR spectroscopy at 639, 1112, 1395, 1616, 1711, and 3423 cm⁻¹.
6. The crystalline quetiapine hemifumarate of clause 5 having an FTIR spectrum substantially as shown in Figure 2.
7. The crystalline quetiapine hemifumarate of clause 1 characterized by a differential scanning calorimetric thermogram with endothermic peaks at about 130°C and at about 166°C.
8. The crystalline quetiapine hemifumarate of clause 7 having a DSC thermogram substantially as shown in figure 3.
9. The crystalline form of quetiapine hemifumarate of clause 1 that is quetiapine hemifumarate chloroform solvate.
10. The crystalline form of quetiapine hemifumarate of clause 1 that is quetiapine hemifumarate methylene chloride solvate.
11. The crystalline quetiapine hemifumarate of clause 1 wherein the quetiapine hemifumarate is micronized.
12. A method of making crystalline quetiapine hemifumarate having at least one characteristic of form II comprising the steps of:
   a) combining quetiapine hemifumarate and a treating solvent that is chloroform, b) refluxing the combination,
   c) cooling the combination after reflux, and
   d) isolating the crystalline form of quetiapine hemifumarate having at least one characteristic of form II.
13. A method of making crystalline quetiapine hemifumarate having at least one characteristic of form II comprising the steps of:
   a) combining quetiapine hemifumarate and a treating solvent that is methylene chloride,
   b) refluxing the combination,
   c) cooling the combination after reflux, and
   d) isolating the crystalline form of quetiapine hemifumarate having at least one characteristic of form II.
14. The method of either of clauses 12 and 13 wherein the cooling is to a temperature of about room temperature.
15. A method of making crystalline quetiapine hemifumarate having at least one characteristic of form II comprising the steps of:
   a) treating quetiapine hemifumarate with a treating solvent selected from chloroform and methylene chloride, and
   b) isolating the crystalline quetiapine hemifumarate having at least one characteristic of form II.
16. The method of clause 15 wherein the treating is by a reflux method comprising the steps of:
   a) combining quetiapine hemifumarate and treating solvent selected from methylene chloride and chloroform,
   b) refluxing the combination,
   c) cooling the combination after reflux, and
   d) isolating the crystalline quetiapine hemifumarate having at least one characteristic of form II.
17. The method of clause 15 wherein the treating is by a solution method comprising the steps of:
   a) providing a solution of quetiapine hemifumarate in a dipolar aprotic solvent at a dissolution temperature,
   b) combining the solution with a treating solvent selected from chloroform and methylene chloride,
   c) cooling the combination to a temperature of about 20° C or less.
18. The method of clause 16 wherein the dissolution temperature is about 80°C.
19. Crystalline quetiapine hemifumarate form II dichloromethane solvate characterized by x-ray reflections at 7.8°, 11.9°, 12.5°, 15.7°, 23.0°, and 23.4°, ± 0.2° 2θ, absorption bands in FTIR at 639, 1112, 1395, 1616, 1711, and 3423 cm⁻¹, and a thermogram in differential scanning calorimetry having endothermic peaks at about 130°C and about 166°C.
20. Crystalline quetiapine hemifumarate form II chloroform solvate characterized by x-ray reflections at 7.8°, 11.9°, 12.5°, 15.7° 23.0°, and 23.4°, ±0.2° 2θ, absorption bands in FTIR at 639, 1112, 1395, 1616, 1711, and 3423 cm-1 and a thermogram in differential scanning calorimetry having endothermic peaks at about 130°C and about 166°C.
21. A crystalline form of quetiapine hemifumarate having at least one characteristic selected from the group consisting of:
   1) x-ray reflections at about 8.9°, 11.8°, 15.3°, 19.4°, 23.0°, and 23.4°, ±0.2° 2θ,
   2) absorption bands in FTIR spectroscopy at 748, 758, 1402, 1607, 1715, and 2883 cm⁻¹ and
   3) a DSC thermogram with endothermic peaks at about 111°C, about 142°C, and about 167°C.
22. The crystalline form of quetiapine hemifumarate of clause 21 characterized by x-ray reflections at about 8.9°, 11.8°, 15.3°, 19.4°, 23.0°, and 23.4°, ±0.2° 2θ.
23. The crystalline form of quetiapine hemifumarate of clause 22 further characterized by x-ray reflections at 16.0°, 17.0°, 17.7°, 18.6°, 20.3°, 20.8°, 21.3°, 21.6°, 26.7°, and 27.4°, ± 0.2° 2θ.
24. The crystalline form of quetiapine hemifumarate of clause 23 having an x-ray diffraction diagram substantially as shown in figure 6.
25. The crystalline form of quetiapine hemifumarate of clause 21 having absorption bands in FTIR spectroscopy at 748, 758, 1402, 1607, 1715, and 2883 cm⁻¹.
26. The crystalline form of quetiapine hemifumarate of clause 25 having an FTIR spectrum substantially as shown in figure 7.
27. The quetiapine hemifumarate of clause 21 having a DSC thermogram with endothermic peaks at about 111°C, about 142°C, and about 167°C.
28. The quetiapine hemifumarate of clause 27 wherein the DSC thermogram is substantially as shown in figure 8.
29. The crystalline form of quetiapine hemifumarate of clause 21 that is quetiapine hemifumarate chloroform solvate.
30. The quetiapine hemifumarate of clause 21 wherein the quetiapine hemifumarate is micronized.
31. A method of making quetiapine hemifumarate having at least one characteristic of form III comprising the steps of:
   a) providing a combination of quetiapine hemifumarate and a dipolar aprotic solvent at a temperature of about 80°C,
   b) mixing the combination with chloroform,
   c) cooling the resulting mixture, and
   d) isolating the quetiapine hemifumarate having at least one characteristic of form III from the mixture.
32. The method of clause 31 wherein the mixture is maintained, with agitation, for a holding time.
33. The method of clause 31 wherein the holding time is at least about 14 hours.
34. Crystalline quetiapine hemifumarate form III chloroform solvate characterized by x-ray reflections at about 8.9°, 11.8°, 15.3°, 19.4°, 23.0°, and 23.4°, ±0.2° 2θ, and absorption bands in FTIR at 748, 758, 1402, 1607, 1715, and 2883 cm⁻¹.
35. A pharmaceutical composition comprising crystalline quetiapine hemifumarate according to either of clauses 1 or 21.
36. The pharmaceutical composition of clause 35 comprising at least one pharmaceutically acceptable excipient.
37. A method of treatment comprising administering to a mammal the pharmaceutical composition including one or more of quetiapine hemifumarate form II chloroform solvate, quetiapine hemifumarate form II dichloromethane solvate and quetiapine hemifumarate form III chloroform solvate, and at least one pharmaceutically acceptable excipient.
38. A crystalline form of quetiapine hemifumarate characterized by x-ray reflections at 11.9°, 12.5°, 14.6°, 15.7°, and 16.8°, ± 0.2° 2θ.
39. A crystalline form of quetiapine hemifumarate characterized by x-ray reflections at 8.9°, 11.8°, 15.3°, 19.4°, 23.0°, and 23.4°, ± 0.2° 2θ.

## Claims

1. A method of making crystalline quetiapine hemifumarate form I comprising the steps of:
a) providing a solution at about 80°C of quetiapine hemifumarate in a solvent selected from the group consisting of water, alkanol, and dipolar aprotic solvents,
b) combining the solution with an anti-solvent whereby a suspension is obtained, and
c) isolating quetiapine hemifumarate form I from the suspension.

2. The method of claim 1 wherein the solvent is an alkanol and the anti-solvent is selected from the group consisting of ethylacetate, isopropylacetate, acetone, methyl tert-butyl ether (MTBE), and acetonitrile.

3. The method of claim 2 wherein the alkanol is isopropyl alcohol or methanol.

4. The method of claim 3 wherein the solvent is a dipolar aprotic solvent selected from the group consisting of dimethylsulfoxide, dimethylformamide, dimethylacetamide and 1-methyl-2-pyrrolidone and the anti-solvent is selected from the group consisting of water, ethylacetate, dichloromethane, toluene, acetone, acetonitrile, isobutanol, ethylacetate, isopropylacetate and methyl tert-butyl ether.

5. A method of making crystalline quetiapine hemifumarate form I comprising the steps of:
a) providing a solution at about 80°C of quetiapine hemifumarate in a solvent selected from the group consisting of alkanol, and a combination of a dipolar aprotic solvent and water,
b) cooling the solution to a temperature of about 20°C or less, and
c) isolating the quetiapine hemifumarate form I from the mixture.

6. The method of claim 5 wherein the alkanol is isopropyl alcohol.

7. The method of claim 5 wherein the dipolar aprotic solvent is dimethylformamide.

8. The method of either of claims 1 or 5 further comprising the steps of post-treating the isolated quetiapine hemifumarate form I by a post-treating method selected from a post-suspension method and a post-recrystallization method.

9. The method of claim 8 wherein the post-treatment method is post suspension comprising the steps of:
a) combining the isolated quetiapine hemifumarate form I with a post-suspending solvent selected from dialkyl ketones, aromatic hydrocarbons, cyanoalkanes, dialkyl ethers, and methylene chloride,
b) refluxing the combination for a reflux time,
c) cooling the combination to ambient temperature, and
d) isolating quetiapine hemifumarate form I.

10. The method of claim 9 further comprising the step of, after cooling of the combination, agitating the cooled combination for an agitating time.

11. The method of claim 9 wherein the post-suspending solvent is selected from the group consisting of acetone, toluene, acetonitrile, dichloromethane, and methyl t-butyl ether.

12. The method of claim 8 wherein the post-treatment method is post-crystallization comprising the steps of:
a) refluxing a solution of the isolated quetiapine hemifumarate form I in a post-crystallization solvent selected from lower alkanols, cyclic ethers, ethyl acetate, and water for a reflux time,
b) cooling the solution to ambient temperature whereby a suspension is formed, and
c) isolating the quetiapine hemifumarate form I.

13. The method of claim 12 further comprising the step of agitating the suspension from step b) at ambient temperature for an agitation time.

14. The method of claim 12 wherein the post-crystallization solvent is selected from the group consisting of water, ethanol, isopropanol, 1-propanol, 1-butanol, 2-butanol, ethyl acetate, tetrahydrofuran, and 1,4-dioxane.
